# EUROPEAN PATENT APPLICATION

(11) **EP 3 037 824 A1**
(43) Date of publication of application: **29.06.2016**
(21) Application number: 14199682.7
(22) Date of filing: 22.12.2014
(51) Int. Cl.: G01N 33/68

(54) **Method for the detection and/or quantification of afamin**

(71) Applicant: Vitateq Biotechnology GmbH, 6020 Innsbruck (AT)
(72) Inventor: DIEPLINGER, Hans, 6020 Innsbruck (AT)
(74) Representative: Sonn & Partner Patentanwälte

(57) **Abstract**

The invention relates to a method for the detection and/or quantification of afamin in a sample comprising
• providing a carrier surface with an immobilized first antibody against afamin,
• contacting a sample which potentially comprises afamin with the first antibody,
• providing a second antibody against afamin
• detecting binding events of afamin, wherein the second antibody has the means to allow detection and/or quantification of binding events by visual inspection and/or spectrophotometry,
characterised in that the first and the second antibody are monoclonal antibodies and bind to different epitopes of afamin.

SEQUENCE LISTING

<110> VITATEQ BIOTECHNOLOGY GMBH

<120> Method for the detection and/or quantification of afamin

<130> R66465

<160> 48

<170> BiSSAP 1.3

<210> 1
<211> 599
<212> PRT
<213> Homo sapiens

<400> 1
Met Lys Leu Leu Lys Leu Thr Gly Phe Ile Phe Phe Leu Phe Phe Leu
1               5                   10                  15
Thr Glu Ser Leu Thr Leu Pro Thr Gln Pro Arg Asp Ile Glu Asn Phe
            20                  25                  30
Asn Ser Thr Gln Lys Phe Ile Glu Asp Asn Ile Glu Tyr Ile Thr Ile
        35                  40                  45
Ile Ala Phe Ala Gln Tyr Val Gln Glu Ala Thr Phe Glu Glu Met Glu
    50                  55                  60
Lys Leu Val Lys Asp Met Val Glu Tyr Lys Asp Arg Cys Met Ala Asp
65                  70                  75                  80
Lys Thr Leu Pro Glu Cys Ser Lys Leu Pro Asn Asn Val Leu Gln Glu
                85                  90                  95
Lys Ile Cys Ala Met Glu Gly Leu Pro Gln Lys His Asn Phe Ser His
            100                 105                 110
Cys Cys Ser Lys Val Asp Ala Gln Arg Arg Leu Cys Phe Phe Tyr Asn
        115                 120                 125
Lys Lys Ser Asp Val Gly Phe Leu Pro Pro Phe Pro Thr Leu Asp Pro
    130                 135                 140
Glu Glu Lys Cys Gln Ala Tyr Glu Ser Asn Arg Glu Ser Leu Leu Asn
145                 150                 155                 160
His Phe Leu Tyr Glu Val Ala Arg Arg Asn Pro Phe Val Phe Ala Pro
                165                 170                 175
Thr Leu Leu Thr Val Ala Val His Phe Glu Glu Val Ala Lys Ser Cys
            180                 185                 190
Cys Glu Glu Gln Asn Lys Val Asn Cys Leu Gln Thr Arg Ala Ile Pro
        195                 200                 205
Val Thr Gln Tyr Leu Lys Ala Phe Ser Ser Tyr Gln Lys His Val Cys
    210                 215                 220
Gly Ala Leu Leu Lys Phe Gly Thr Lys Val Val His Phe Ile Tyr Ile
225                 230                 235                 240
Ala Ile Leu Ser Gln Lys Phe Pro Lys Ile Glu Phe Lys Glu Leu Ile
                245                 250                 255
Ser Leu Val Glu Asp Val Ser Ser Asn Tyr Asp Gly Cys Cys Glu Gly
            260                 265                 270
Asp Val Val Gln Cys Ile Arg Asp Thr Ser Lys Val Met Asn His Ile
        275                 280                 285
Cys Ser Lys Gln Asp Ser Ile Ser Ser Lys Ile Lys Glu Cys Cys Glu
    290                 295                 300
Lys Lys Ile Pro Glu Arg Gly Gln Cys Ile Ile Asn Ser Asn Lys Asp
305                 310                 315                 320
Asp Arg Pro Lys Asp Leu Ser Leu Arg Glu Gly Lys Phe Thr Asp Ser
                325                 330                 335
Glu Asn Val Cys Gln Glu Arg Asp Ala Asp Pro Asp Thr Phe Phe Ala
            340                 345                 350
Lys Phe Thr Phe Glu Tyr Ser Arg Arg His Pro Asp Leu Ser Ile Pro

355                 360                 365
Glu Leu Leu Arg Ile Val Gln Ile Tyr Lys Asp Leu Leu Arg Asn Cys
    370                 375                 380
Cys Asn Thr Glu Asn Pro Pro Gly Cys Tyr Arg Tyr Ala Glu Asp Lys
385                 390                 395                 400
Phe Asn Glu Thr Thr Glu Lys Ser Leu Lys Met Val Gln Gln Glu Cys
                405                 410                 415
Lys His Phe Gln Asn Leu Gly Lys Asp Gly Leu Lys Tyr His Tyr Leu
            420                 425                 430
Ile Arg Leu Thr Lys Ile Ala Pro Gln Leu Ser Thr Glu Glu Leu Val
        435                 440                 445
Ser Leu Gly Glu Lys Met Val Thr Ala Phe Thr Thr Cys Cys Thr Leu
    450                 455                 460
Ser Glu Glu Phe Ala Cys Val Asp Asn Leu Ala Asp Leu Val Phe Gly
465                 470                 475                 480
Glu Leu Cys Gly Val Asn Glu Asn Arg Thr Ile Asn Pro Ala Val Asp
                485                 490                 495
His Cys Cys Lys Thr Asn Phe Ala Phe Arg Arg Pro Cys Phe Glu Ser
            500                 505                 510
Leu Lys Ala Asp Lys Thr Tyr Val Pro Pro Pro Phe Ser Gln Asp Leu
        515                 520                 525
Phe Thr Phe His Ala Asp Met Cys Gln Ser Gln Asn Glu Glu Leu Gln
    530                 535                 540
Arg Lys Thr Asp Arg Phe Leu Val Asn Leu Val Lys Leu Lys His Glu
545                 550                 555                 560
Leu Thr Asp Glu Glu Leu Gln Ser Leu Phe Thr Asn Phe Ala Asn Val
                565                 570                 575
Val Asp Lys Cys Cys Lys Ala Glu Ser Pro Glu Val Cys Phe Asn Glu
            580                 585                 590
Glu Ser Pro Lys Ile Gly Asn
        595

<210> 2
<211> 15
<212> PRT
<213> Homo sapiens

<400> 2
Arg Leu Cys Phe Phe Tyr Asn Lys Lys Ser Asp Val Gly Phe Leu
1               5                   10                  15

<210> 3
<211> 15
<212> PRT
<213> Homo sapiens

<400> 3
Phe Tyr Asn Lys Lys Ser Asp Val Gly Phe Leu Pro Pro Phe Pro
1               5                   10                  15

<210> 4
<211> 15
<212> PRT
<213> Homo sapiens

<400> 4
Asn Arg Glu Ser Leu Leu Asn His Phe Leu Tyr Glu Val Ala Arg
1               5                   10                  15

<210> 5
<211> 15
<212> PRT
<213> Homo sapiens

<400> 5
Leu Leu Asn His Phe Leu Tyr Glu Val Ala Arg Arg Asn Pro Phe
1               5                   10                  15

<210> 6
<211> 15
<212> PRT
<213> Homo sapiens

<400> 6
Phe Leu Tyr Glu Val Ala Arg Arg Asn Pro Phe Val Phe Ala Pro
1               5                   10                  15

<210> 7
<211> 15
<212> PRT
<213> Homo sapiens

<400> 7
Val Ala Arg Arg Asn Pro Phe Val Phe Ala Pro Thr Leu Leu Thr
1               5                   10                  15

<210> 8
<211> 15
<212> PRT
<213> Homo sapiens

<400> 8
Asn Pro Phe Val Phe Ala Pro Thr Leu Leu Thr Val Ala Val His
1               5                   10                  15

<210> 9
<211> 15
<212> PRT
<213> Homo sapiens

<400> 9
Phe Ala Pro Thr Leu Leu Thr Val Ala Val His Phe Glu Glu Val
1               5                   10                  15

<210> 10
<211> 15
<212> PRT
<213> Homo sapiens

<400> 10
Leu Gln Thr Arg Ala Ile Pro Val Thr Gln Tyr Leu Lys Ala Phe
1               5                   10                  15

<210> 11
<211> 15
<212> PRT
<213> Homo sapiens

<400> 11
Ala Ile Pro Val Thr Gln Tyr Leu Lys Ala Phe Ser Ser Tyr Gln
1               5                   10                  15

<210> 12
<211> 15
<212> PRT
<213> Homo sapiens

<400> 12
Ala Leu Leu Lys Phe Gly Thr Lys Val Val His Phe Ile Tyr Ile
1               5                   10                  15

<210> 13
<211> 15
<212> PRT
<213> Homo sapiens

<400> 13
Phe Gly Thr Lys Val Val His Phe Ile Tyr Ile Ala Ile Leu Ser
1               5                   10                  15

<210> 14
<211> 15
<212> PRT
<213> Homo sapiens

<400> 14
Val Val His Phe Ile Tyr Ile Ala Ile Leu Ser Gln Lys Phe Pro
1               5                   10                  15

<210> 15
<211> 15
<212> PRT
<213> Homo sapiens

<400> 15
Ile Tyr Ile Ala Ile Leu Ser Gln Lys Phe Pro Lys Ile Glu Phe
1               5                   10                  15

<210> 16
<211> 15
<212> PRT
<213> Homo sapiens

<400> 16
Ile Leu Ser Gln Lys Phe Pro Lys Ile Glu Phe Lys Glu Leu Ile
1               5                   10                  15

<210> 17
<211> 15
<212> PRT
<213> Homo sapiens

<400> 17
Lys Phe Pro Lys Ile Glu Phe Lys Glu Leu Ile Ser Leu Val Glu
1               5                   10                  15

<210> 18
<211> 15
<212> PRT
<213> Homo sapiens

<400> 18
Glu Arg Asp Ala Asp Pro Asp Thr Phe Phe Ala Lys Phe Thr Phe
1               5                   10                  15

<210> 19
<211> 15
<212> PRT
<213> Homo sapiens

<400> 19
Asp Pro Asp Thr Phe Phe Ala Lys Phe Thr Phe Glu Tyr Ser Arg
1               5                   10                  15

<210> 20
<211> 15
<212> PRT
<213> Homo sapiens

<400> 20
Phe Phe Ala Lys Phe Thr Phe Glu Tyr Ser Arg Arg His Pro Asp
1               5                   10                  15

<210> 21
<211> 15
<212> PRT
<213> Homo sapiens

<400> 21
Phe Thr Phe Glu Tyr Ser Arg Arg His Pro Asp Leu Ser Ile Pro
1               5                   10                  15

<210> 22
<211> 15
<212> PRT
<213> Homo sapiens

<400> 22
Tyr Ser Arg Arg His Pro Asp Leu Ser Ile Pro Glu Leu Leu Arg
1               5                   10                  15

<210> 23
<211> 15
<212> PRT
<213> Homo sapiens

<400> 23
His Pro Asp Leu Ser Ile Pro Glu Leu Leu Arg Ile Val Gln Ile
1               5                   10                  15

<210> 24
<211> 15
<212> PRT
<213> Homo sapiens

<400> 24
Ser Ile Pro Glu Leu Leu Arg Ile Val Gln Ile Tyr Lys Asp Leu
1               5                   10                  15

<210> 25
<211> 15
<212> PRT
<213> Homo sapiens

<400> 25
His Phe Gln Asn Leu Gly Lys Asp Gly Leu Lys Tyr His Tyr Leu
1               5                   10                  15

<210> 26
<211> 15
<212> PRT
<213> Homo sapiens

<400> 26
Leu Gly Lys Asp Gly Leu Lys Tyr His Tyr Leu Ile Arg Leu Thr
1               5                   10                  15

<210> 27
<211> 15
<212> PRT
<213> Homo sapiens

<400> 27
Gly Leu Lys Tyr His Tyr Leu Ile Arg Leu Thr Lys Ile Ala Pro
1               5                   10                  15

<210> 28
<211> 15
<212> PRT
<213> Homo sapiens

<400> 28
His Tyr Leu Ile Arg Leu Thr Lys Ile Ala Pro Gln Leu Ser Thr
1               5                   10                  15

<210> 29
<211> 15
<212> PRT
<213> Homo sapiens

<400> 29
Glu Leu Gln Arg Lys Thr Asp Arg Phe Leu Val Asn Leu Val Lys
1               5                   10                  15

<210> 30
<211> 15
<212> PRT
<213> Homo sapiens

<400> 30
Lys Thr Asp Arg Phe Leu Val Asn Leu Val Lys Leu Lys His Glu
1               5                   10                  15

<210> 31
<211> 15
<212> PRT
<213> Homo sapiens

<400> 31
Phe Leu Val Asn Leu Val Lys Leu Lys His Glu Leu Thr Asp Glu
1               5                   10                  15

<210> 32
<211> 15
<212> PRT
<213> Homo sapiens

<400> 32
Arg Leu Cys Phe Phe Tyr Asn Lys Lys Ser Asp Val Gly Phe Leu
1               5                   10                  15

<210> 33
<211> 15
<212> PRT
<213> Homo sapiens

<400> 33
Phe Tyr Asn Lys Lys Ser Asp Val Gly Phe Leu Pro Pro Phe Pro
1               5                   10                  15

<210> 34
<211> 15
<212> PRT
<213> Homo sapiens

<400> 34
Phe Leu Tyr Glu Val Ala Arg Arg Asn Pro Phe Val Phe Ala Pro
1               5                   10                  15

<210> 35
<211> 15
<212> PRT
<213> Homo sapiens

<400> 35
Val Ala Arg Arg Asn Pro Phe Val Phe Ala Pro Thr Leu Leu Thr
1               5                   10                  15

<210> 36
<211> 15
<212> PRT
<213> Homo sapiens

<400> 36
Leu Gln Thr Arg Ala Ile Pro Val Thr Gln Tyr Leu Lys Ala Phe
1               5                   10                  15

<210> 37
<211> 15
<212> PRT
<213> Homo sapiens

<400> 37
Ala Ile Pro Val Thr Gln Tyr Leu Lys Ala Phe Ser Ser Tyr Gln
1               5                   10                  15

<210> 38
<211> 15
<212> PRT
<213> Homo sapiens

<400> 38
Val Val His Phe Ile Tyr Ile Ala Ile Leu Ser Gln Lys Phe Pro
1               5                   10                  15

<210> 39
<211> 15
<212> PRT
<213> Homo sapiens

<400> 39
Ile Tyr Ile Ala Ile Leu Ser Gln Lys Phe Pro Lys Ile Glu Phe
1               5                   10                  15

<210> 40
<211> 15
<212> PRT
<213> Homo sapiens

<400> 40
Ile Leu Ser Gln Lys Phe Pro Lys Ile Glu Phe Lys Glu Leu Ile
1               5                   10                  15

<210> 41
<211> 15
<212> PRT
<213> Homo sapiens

<400> 41
Asn His Ile Cys Ser Lys Gln Asp Ser Ile Ser Ser Lys Ile Lys
1               5                   10                  15

<210> 42
<211> 15
<212> PRT
<213> Homo sapiens

<400> 42
Asp Pro Asp Thr Phe Phe Ala Lys Phe Thr Phe Glu Tyr Ser Arg
1               5                   10                  15

<210> 43
<211> 15
<212> PRT
<213> Homo sapiens

<400> 43
Phe Phe Ala Lys Phe Thr Phe Glu Tyr Ser Arg Arg His Pro Asp
1               5                   10                  15

<210> 44
<211> 15
<212> PRT
<213> Homo sapiens

<400> 44
Tyr Ser Arg Arg His Pro Asp Leu Ser Ile Pro Glu Leu Leu Arg
1               5                   10                  15

<210> 45
<211> 15
<212> PRT
<213> Homo sapiens

<400> 45
Gly Leu Lys Tyr His Tyr Leu Ile Arg Leu Thr Lys Ile Ala Pro
1               5                   10                  15

<210> 46
<211> 15
<212> PRT
<213> Homo sapiens

<400> 46
His Tyr Leu Ile Arg Leu Thr Lys Ile Ala Pro Gln Leu Ser Thr
1               5                   10                  15

<210> 47
<211> 15
<212> PRT
<213> Homo sapiens

<400> 47
Glu Leu Gln Arg Lys Thr Asp Arg Phe Leu Val Asn Leu Val Lys
1               5                   10                  15

<210> 48
<211> 15
<212> PRT
<213> Homo sapiens

<400> 48
Lys Thr Asp Arg Phe Leu Val Asn Leu Val Lys Leu Lys His Glu
1               5                   10                  15

## Description

The present invention relates to the field of afamin diagnostics in humans.

Afamin is a 87 kDa protein belonging to the albumin group (Lichenstein HS, J Biol Chem 1994; 269:18149-54) and having many things in common, structurally and in terms of biochemistry, with the proteins of this group, such as, e.g., with human serum albumin (HSA), human [alpha]-fetoprotein (AFP) or human vitamin D binding protein. Afamin has already been cloned and sequenced and thus is also available in recombinant form (WO 95/27059 A). Afamin is a glycoprotein primarily expressed in the liver and is secreted into the circulation. It has been shown that afamin occurs abundantly in plasma. Minor expressions have been described also in human brain (Kratzer I et al. J Neurochem 2009; 108:707-18), heart, kidney, testis and ovary (www.proteinatlas.org). Afamin has been reported to bind vitamin E, especially α-Tocopherol and γ-Tocopherol, two of the most important forms of vitamin E, in vivo and to possess multiple binding sites for both tocopherol isomers (Jerkovic L et al. J Proteome Res 2005; 4:889-99 and Voegele AF et al. Biochemistry 2002; 41:14532-8). Apart from its sequence homologies to albumin, little is known about the function of afamin.

Comparative proteomics has previously identified afamin as a potential biomarker for ovarian cancer (Jackson D et al. Clin Cancer Res 2007; 13:7370-9). This finding was confirmed with immunoblotting and quantitative immunoassay for afamin. Patients with ovarian cancer displayed significantly decreased plasma concentrations of afamin by comparison to healthy controls. These results were later validated in an independent larger study of patients with ovarian cancer (Dieplinger H et al. Cancer Epidemiol Biomarkers Prev 2009; 18:1127-33) and, recently, extended by showing significant associations between afamin plasma concentrations and clinical outcomes (response to therapy and survival rates) (Melmer A et al. Gynecol Oncol 2013; 128:48-43). In contrast, afamin concentrations were not found to be decreased in benign gynaecological conditions including endometriosis (Dieplinger H et al. Cancer Epidemiol Biomarkers Prev 2009; 18:1127-33 and Seeber BE et al. Fertil Steril 2010; 94:2923-6).

Afamin has been reported in three large, independent population-based studies to be highly significantly associated with the prevalence and incidence of metabolic syndrome (Kronenberg F et al. Circ Cardiovasc Gene 2014). In line with these findings, elevated afamin concentrations have also been described for pregnant women suffering from pregnancy complications such as gestational diabetes and preeclampsia and patent with polycystic ovary syndrome (Hubalek M et al. Clin Chim Acta 2014 2014; 434:41-7 and Koninger A et al. Endocrine connections 2014; 3:120-6). These conditions have close similarities with features of the metabolic syndrome and related clinical manifestations.

Accordingly, the diagnostics of afamin in the human body by means of identification and/or quantification is of great importance. Afamin quantification in various body fluids as a marker for certain diseases has been disclosed e.g. in WO 2001/001148 A, WO 2002/050549 A, WO 2002/087604 A, WO 2006/079136 A, WO 2009/029971 A, WO 2010/037152 A and WO 2013/000992 A. Recently, a fully automated immunoassay for the quantitative measurement of afamin in human plasma was reported (Dieplinger B et al. Clin Chim Acta 2013; 425:236:41). In this study, a sandwich enzyme-linked immunosorbent assay (ELISA) for afamin was tested. The ELISA used an affinity-purified polyclonal rabbit anti-human afamin antibody for coating 96-well microtiter plates and an enzyme-conjugated monoclonal mouse anti-human afamin antibody, which was conjugated with horse-radish peroxidase for detection. As the above-mentioned method for the detection of afamin, other disclosed methods for the detection of afamin (e.g. WO 2009/029971 A, WO 2013000992 A) use at least one polyclonal antibody against afamin. A problem of polyclonal antibodies is that they are prone to batch-to-batch variability and can give background signals as the antibodies are non-specific. Further, polyclonal antibodies can exhibit cross-reactivity with other proteins. Therefore, methods for the detection of afamin using polyclonal antibodies can be limited in the standardization and reproducibility of test results and in the specificity in regard to the protein of interest.

It is therefore an object of the present invention to provide a method for the detection and/or quantification of afamin in a sample with high specificity and high reproducibility.

It was now surprisingly found in the course of the present invention that a method using two monoclonal antibodies which bind to different epitopes of afamin has a beneficial specificity and reproducibility. Further, it was surprisingly found in the present invention that the specific arrangement of the two monoclonal antibodies is essential for the performance of the method.

The present invention provides therefore in a first aspect a method for the detection and/or quantification of afamin in a sample comprising
- providing a carrier surface with an immobilized first antibody against afamin,
- contacting a sample which potentially comprises afamin with the first antibody,
- providing a second antibody against afamin
- detecting binding events of afamin, wherein the second antibody has the means to allow detection and/or quantification of binding events by visual inspection and/or spectrophotometry,
characterised in that the first and the second antibody are monoclonal antibodies and bind to different epitopes of afamin.

The term "antibody" characterises in connection with the present invention a domain of a polypeptide which specifically binds/interacts with a given target structure/antigen/epitope. The target structure in light of the present invention is afamin and epitopes of afamin accordingly. Further, the term "antibody" also comprises derivatives or functional fragments thereof which still retain the binding specificity to afamin. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also comprises immunogloblulins (Ig's) of different classes (i.e. IgA, IgG, IgM, IgD and IgE) and subclasses (such as IgG1, IgG2 etc.). The definition of the term "antibody" also includes embodiments such as chimeric and single chain antibodies, as well as antibody fragments, like, inter alia, Fab fragments. Antibody fragments or derivatives further comprise F(ab')₂, Fv, scFv fragments or single domain antibodies, single variable domain antibodies or immunoglobulin single variable domain comprising merely one variable domain, which might be V_{H} or V_{L}, that specifically bind afamin or an epitope of afamin independently of other V regions or domains. Such immunoglobulin single variable domain encompasses not only an isolated antibody single variable domain polypeptide, but also larger polypeptides that comprise one or more monomers of an antibody single variable domain polypeptide sequence.

Various procedures are known in the art and may be used for the production of such antibodies and/or fragments thereof. Thus, the (antibody) derivatives can be produced by peptidomimetics. Further, techniques described for the production of single chain antibodies (see i.a. US Patent 4,946,778) can be adapted to produce single chain antibodies specific for elected polypeptide(s). Also transgenic animals, such as mice, may be used to express humanized antibodies specific for polypeptides and fusion proteins of this invention. For the preparation of monoclonal antibodies, any technique, providing antibodies produced by continuous cell line cultures can be used. Examples for such techniques include the hybridoma technique (Köhler and Milstein, Nature 1975. 256:495-497), the trioma technique, the human B-cell hybridoma technique (Kozbor and Roder, Immunology Today 1983. 4:72) and the EBV-hybridoma technique to produce human monoclonal antibodies (Cole et al. Monoclonal Antibodies and Cancer Therapy, Alan R. Liss, Inc. 1985. 77-96). It is also envisaged in the context of this invention that the term "antibody" comprises antibody constructs, which may be expressed in a host as described herein, e.g. antibody constructs which may be transfected and/or transduced via, inter alia, viruses or plasmid vectors.

The term "epitope" defines a binding determinant of afamin, which is specifically bound/identified by an antibody as defined above. The antibodies may specifically bind to/interact with conformational or continuous epitopes, which are unique for the target structure, e.g. the human afamin. A conformational or discontinuous epitope is characterised in the primary sequence, but come together on the surface of the molecule when the polypeptide fold into the native protein (Sela, Science 1969. 166:1365 and Laver, Cell 1990. 61:553-556). The two or more discrete amino acid residues contributing to the epitope are present on separate sections of one or more polypeptide chain(s). These residues come together on the surface of the molecule when the polypeptide chain(s) fold(s) into a three-dimensional structure to constitute the epitope. In contrast, a continuous or linear epitope consists of two or more discrete amino acid residues, which are present in a single linear segment of a polypeptide chain.

According to the invention, these two monoclonal antibodies bind to different epitopes of the afamin. In a preferred embodiment of the invention, the two monoclonal antibodies bind to different conformational epitopes.

"Different" in the context of the present invention means that the amino acids of afamin that are recognized by the antibodies differ by at least 10 amino acids, preferably by 20 amino acids, preferably by 30 amino acids, more preferably by 40 amino acids, even more preferably by 50 amino acids, most preferred by 59 amino acids.

The epitopes recognized by the antibodies can be determined by the PepSpot blotting technique. In an embodiment of the present invention, the first and the second monoclonal antibodies are able to recognize the amino acids listed in table 1 of the application. In an embodiment of the present invention, the first or second antibody binds to an epitope which includes amino acids Arg155, Leu227, and Phe419. In a preferred embodiment of the present invention, the first antibody binds to an epitope which includes amino acids Arg155, Leu227, and Phe419. In another embodiment of the present invention, the first or second antibody binds to an epitope which includes amino acids Leu202, Ile288, and Lys300. In a preferred embodiment of the present invention, the second antibody binds to an epitope which includes amino acids Leu202, Ile288, and Lys300. In a preferred embodiment of the present invention, the first antibody binds to an epitope which includes amino acids Arg155, Leu227, and Phe419 and the second antibody binds to an epitope which includes amino acids Leu202, Ile288, and Lys300.

According to the present invention, the first antibody is immobilized on a carrier surface. The immobilization of the first antibody on the carrier surface can be performed by any means known in the art. In an embodiment of the present invention, the first antibody is immobilized on the carrier surface by adhesion or by biotin/streptavidin coupling. In a preferred embodiment of the present invention, the first antibody is immobilized on the carrier surface by adhesion.

The carrier surface can be any surface suitable for the above mentioned method and can be used for the inventive detection and/or quantification of afamin. Such a carrier surface is preferably a solid surface, a semi-solid surface such as a gel, or a membrane. The carrier surface is preferably inert and/or passivated to prevent unspecific binding of the sample content. In further embodiment of the present invention, the carrier surface exhibits wells or microwells suitable for uptake of solutions e.g. sample solutions, buffer solutions or chromogenic substrate solutions. In a preferred embodiment of the present invention, the carrier is a microtiter plate.

In an aspect of the present invention, the second antibody has the means to allow detection and/or quantification of binding events by visual inspection and/or spectrophotometry. In an embodiment of the present invention, this means comprise an enzyme, a fluorophore or a dye bound to the second antibody. Accordingly, the second antibody is an enzyme-labelled, fluorescence-labelled or dye-labelled antibody. In a preferred embodiment of the present invention, the second antibody is an enzyme-labelled antibody. The enzyme bound to the antibody is preferably horse-radish peroxidase, glucose oxidase or alkaline phosphatase. In a preferred embodiment of the present invention, the enzyme is horse-radish peroxidase.

In another aspect of the present invention, the binding event can be detected by an additional, third, antibody which is specific for the Fc portion of the second or first antibody of the present invention. In this aspect of the present invention, said third antibody is labelled, preferably enzyme labelled, fluorescence labelled or dye labelled. The enzyme can be horse-radish peroxidase, glucose oxidase or alkaline phosphatase, preferably horse-radish peroxidase. The first or second antibody contains constant portions (Fc) which can be uniformly targeted by the third antibody. It is thus possible to detect the first or second antibodies bound to afamin by using third antibodies which bind to these antibodies and then generate a signal, which is directly proportional to the first or second antibodies bound to afamin.

In an embodiment of the present invention, a substrate solution is provided to the inventive method. This substrate solution comprises a chromogenic substrate for the used enzyme bound to the second or third antibody. In a preferred embodiment of the invention, the chromogenic substrate solution comprises a chromogenic substrate for the horse-radish peroxidase enzyme. In another preferred embodiment of the invention, the chromogenic substrate is 3,3',5,5'-tetramethylbenzidine.

In an embodiment of the present invention, the sample which potentially comprises afamin and wherein the afamin is detected is a sample of the human body, preferably of body fluid or a tissue sample. In a preferred embodiment, the sample is a blood, blood plasma or blood serum sample. The amount detected in the sample is usually expressed relatively to its concentration in a fluid (e.g. as mg afamin/l blood).

According to another aspect of the invention, a kit is provided for performing the method according to the present invention comprising - besides the suitable means of the present invention - a reference means of afamin. In an embodiment of the invention, the reference means is a standardized amount of afamin. This standard amount of afamin can represent the usual afamin concentration in a body fluid or tissue sample of a healthy subject. In another embodiment of the present invention, the reference means is a series of standardized samples of afamin. These standardized samples of afamin can represent different afamin concentrations in a body fluid or tissue sample of a subject. The different afamin concentrations can represent different medical conditions or represent different stages or severity of a medical condition. The different afamin concentrations can also represent different stages of pregnancy in a woman. The comparison of the measured afamin concentration in a sample with a standardized amount of afamin or a series of standardized samples of afamin is thereby indicative for a medical condition, the stage or severity of a medical condition or a stage of pregnancy, if the amount of afamin measured in the sample is equal or in a certain range to a standardized amount of afamin or to one from the standardized samples of the series of afamin. Said certain range can be a deviation of 20%, preferably 15%, more preferably 10%, even more preferably 5% from the standard amount of afamin.

Medical conditions associated with an alteration in the afamin concentration in a sample of the human body are metabolic syndrome; infertility; neurodegenerative diseases like Alzheimer's disease, Huntington's Disease, amyotrophic lateral sclerosis, cerebral ischemia, dementia; epilepsy; Parkinson's Disease; tumours of the reproductive organs like testicular, ovarian tumours or germ cell tumours; gestational diabetes; preeclampsia and polycystic ovary syndrome.

The invention therefore relates in one aspect of the invention to the use of a kit for the comparison of an afamin content in a sample with a reference value.

The present invention is further illustrated by the following figures and examples without being in any way a limitation of the scope of the invention.

### FIGURES

Table 1 shows the afamin peptides recognized by the first and second monoclonal antibodies with PepSpot blotting technique.
Table 2 shows the afamin concentrations measured in the plasma from 72 probands with different ELISA configurations. Configurations from left to right columns: polyclonal antibody immobilized on the carrier surface (coating) and the first monoclonal antibody used for detecting binding events (N13, labelled with horse-radish peroxidase); polyclonal antibody immobilized on the carrier surface (coating) and the second monoclonal antibody used for detecting binding events (M14, labelled with horse-radish peroxidase); second monoclonal antibody immobilized on the carrier surface (M14, coating) and the first monoclonal antibody used for detecting binding events (N13, labelled with horse-radish peroxidase); first monoclonal antibody immobilized on the carrier surface (N13, coating) and the second monoclonal antibody used for detecting binding events (M14, labelled with horse-radish peroxidase).
Table 3 shows the afamin concentrations (mg/l) measured with the inventive ELISA method (first monoclonal antibody immobilized on the carrier surface (N13, coating) and the second monoclonal antibody used for detecting binding events (M14, labelled with horse-radish peroxidase) and the commercially available Uscn ELISA.

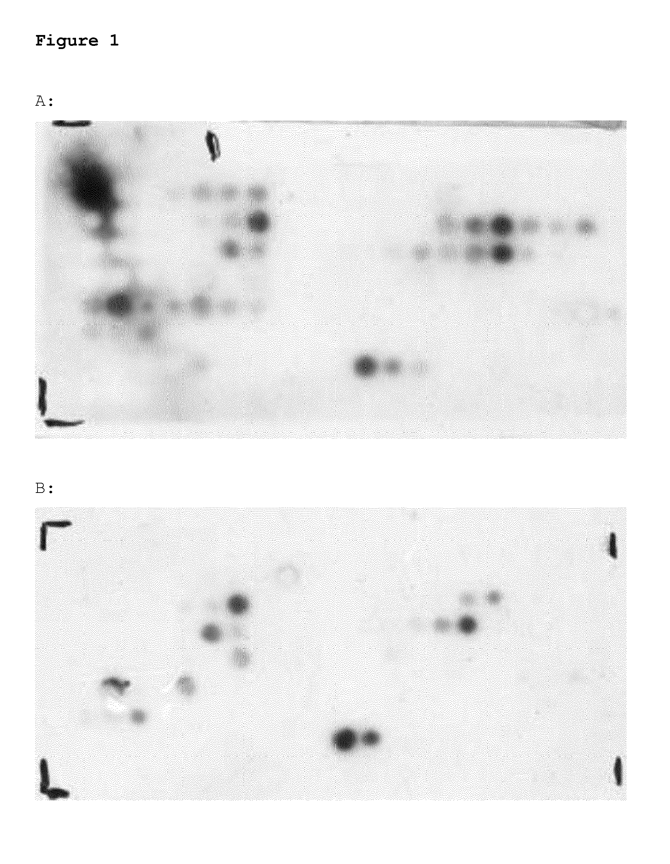

Figure 1 shows the PepSpot analysis of epitope recognition of the first monoclonal antibody (N13, a) and the second monoclonal antibody (M14, b).

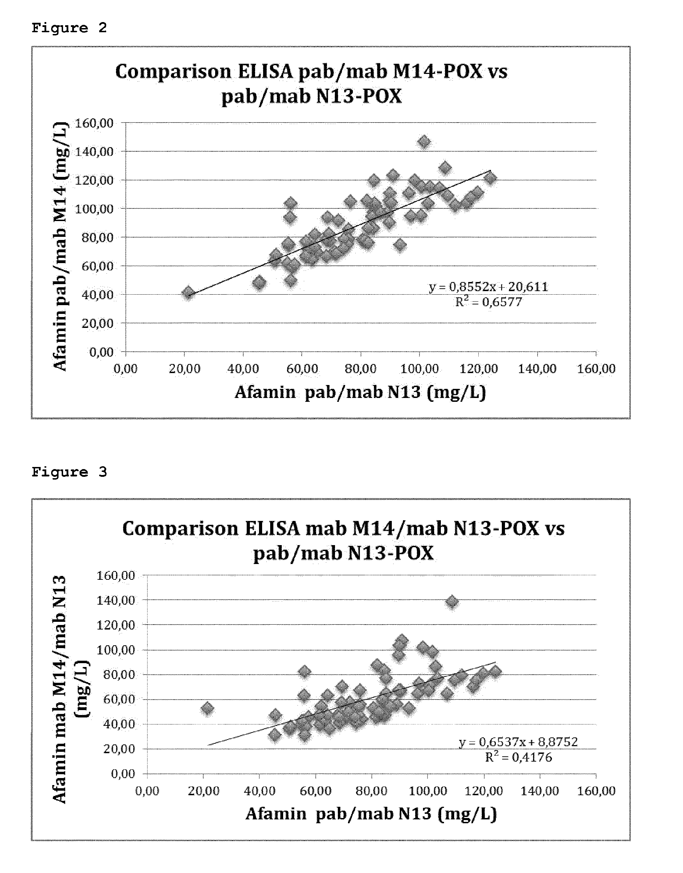

Figure 2 shows the correlation of measured afamin concentrations between the ELISA, wherein the polyclonal antibody is immobilized on the carrier surface (coating) and the second monoclonal antibody is used for detecting binding events (M14, labelled with horse-radish peroxidase), and the ELISA, wherein the polyclonal antibody is immobilized on the carrier surface and the first monoclonal antibody is used for detecting binding events (N13, labelled with horse-radish peroxidase).
Figure 3 shows the correlation of measured afamin concentrations between the ELISA, wherein the second monoclonal antibody is immobilized on the carrier surface (M14, coating) and the first monoclonal antibody is used for detecting binding events (N13, labelled with horse-radish peroxidase), and the ELISA, wherein the polyclonal antibody is immobilized on the carrier surface (coating) and the first monoclonal antibody is used for detecting binding events (N13, labelled with horse-radish peroxidase).

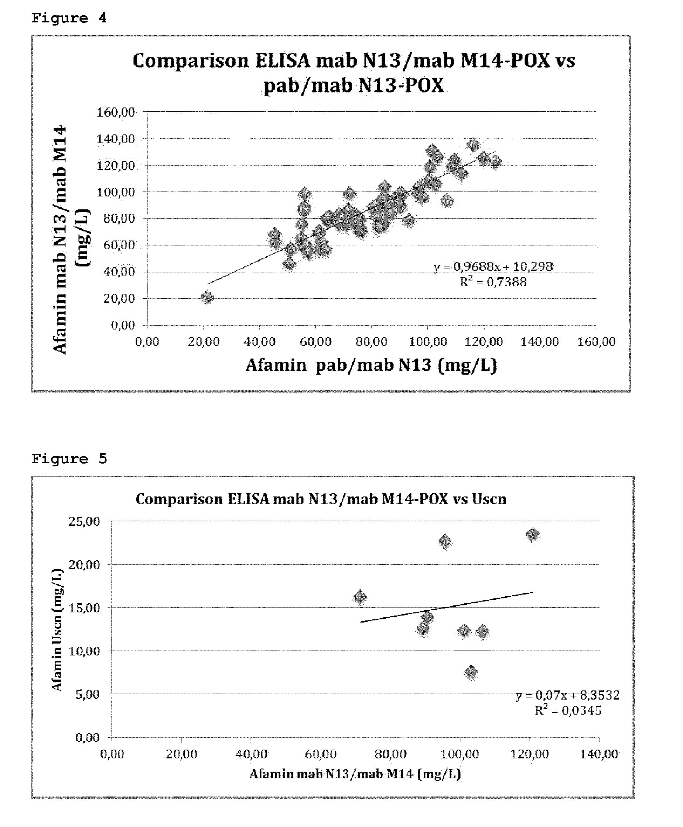

Figure 4 shows the correlation of measured afamin concentrations between the inventive ELISA method, wherein the first monoclonal antibody is immobilized on the carrier surface (N13, coating) and the second monoclonal antibody is used for detecting binding events (M14, labelled with horse-radish peroxidase), and the ELISA, wherein the polyclonal antibody is immobilized on the carrier surface (coating) and the first monoclonal antibody is used for detecting binding events (N13, labelled with horse-radish peroxidase).
Figure 5 shows the correlation of measured afamin concentrations between the inventive ELISA method, wherein the first monoclonal antibody is immobilized on the carrier surface (N13, coating) and the second monoclonal antibody is used for detecting binding events (M14, labelled with horse-radish peroxidase), and the commercially available Uscn ELISA.

### EXAMPLES

### Example 1 Description and Characterization of used antibodies

The custom-made polyclonal rabbit antibody against human afamin was obtained from Gramsch Laboratories, Schwabhausen, Germany, by immunizing New Zealand White Rabbits with human afamin, purified to homogeneity from human plasma using complete Freund's adjuvans (CFA). A specific anti-afamin antibody was prepared from rabbit antiserum by immunoaffinity chromatography using a CNBr-activated sepharose column to which purified human afamin was covalently bound.

The first monoclonal anti-afamin antibody (referred to as mab N13) and the second monoclonal anti-afamin antibody (referred to as mab M14) were obtained with conventional hybridoma technology by immunizing Balb/c mice with purified human afamin, dissolved in PBS. Mice were immunized according to the following protocol: at time 1, 100 microgram/animal intraperitoneal (i.p.) with CFA; at times 1+30, 60, 90 days, 50 microgram/animal i.p. at each time point, with IFA (incomplete Freund's adjuvant); at times 1+120, 121, 122 days, 50 microgram/animal intravenously at each time point, without adjuvant. On day 1+123, the antibody titer was determined from a retro-orbital bleed of each animal by "quick-ELISA" testing. This test was performed by incubating diluted mouse serum in microtiter plates (MTPs), previously coated with 5 microgram/ml of purified human afamin, followed by detection with peroxidase-labeled anti-mouse antibody and appropriate substrate reaction. The animal with the highest antibody titer was used on the same day for splenic fusion and creation of hybridomas. After establishing the hybridomas as stable cell cultures in 96-well culture plates with the help of mouse peritoneal macrophages as feeder cells, supernatants were screened for binding to purified human afamin again by "quick-ELISA" testing. Of the candidate hybridomas producing antibodies with high affinity for afamin, two mabs (N13 and M14) were selected after additional screening by immunoblotting. These two antibodies recognized only one protein band on immunoblotted purified afamin as well as human plasma, indicating high specificity for afamin. Antibody monoclonality was achieved by limiting dilution of hybridoma cells. Selected hybridoma cell lines were first cultivated in RPMI 1640 medium (Gibco, Life Technologies) containing 10% FCS and then transferred to serum- and protein-free culture using PFHM II medium. Under these conditions, approximately 30 mg/L immunoglobulin was found in the serum-free culture medium. Mabs N13 and M14 were purified from serum-free hybridoma cell culture supernatants with affinity chromatography using Protein G-coupled sepharose columns (GE Healthcare, Uppsala, Sweden). Immunoglobulins were isotyped using a commercially available kit (Monoclonal Isotyping Kit, American Qualex, San Clemente, CA, USA).

To characterize in detail binding characteristics of mabs N13 and M14, epitope mapping for the two antibodies was performed using the PepSpots peptide array technology by JPT Peptide Technologies (Berlin, Germany). 142 peptides, composed of 15 overlapping amino acids each, were bound to cellulose membranes and their recognition by antibodies N13 and M14 analysed by dot blotting.

### Example 2 Established ELISA - polyclonal/monoclonal sandwich

Afamin was measured with photometric enzyme-linked immunosorbent assay (ELISA) on an automated Tecan Instrument. The afamin ELISA is a custom-made double-antibody sandwich assay (MicroCoat GmbH, Bernried, Germany) using the affinity-purified polyclonal rabbit anti-human-afamin antibody for coating 96-well MTPs and the enzyme-conjugated monoclonal mouse anti-human afamin antibody N13 for detection.

Mab N13 was conjugated with horse-radish peroxidase (POX) according to standard protocols and purified by size-exclusion chromatography prior to use. The biotinylated coating antibody was bound to streptavidin-coated plates, followed by blocking unspecific binding by incubation with 0.1% casein in phosphate-buffered-saline (PBS). After removing blocking solution MTPs were sealed and kept at 4°C until use.

All subsequent incubation steps were carried out at room temperature (18-25°C). Antibody-coated MTPs were washed three times with 300 µL of washing buffer [Tween 20 (1/2000, vol/vol) in PBS, pH 7.3] and incubated with 100 µL blank, standards, and plasma samples after a standard 1:1000 dilution with assay buffer (0.1% casein in PBS, pH 7.3), washing buffer under constant agitation at 750 rpm for 60 minutes.

A secondary plasma standard with an afamin concentration of 80 mg/L, calibrated with purified human afamin, was used in a serial dilution for the present study. Seven standards with the following final dilutions were used for each assay: S1=0.200 mg/L, S2=0.100 mg/L, S3=0.050 mg/L, S4=0.025 mg/L, S5=0.0125 mg/L, S6=0.0063 mg/L, S7=0.0031 mg/L.

MTPs were then washed three times with 300 µL washing buffer and incubated with 100 µL horse-radish-peroxidase-conjugated monoclonal antibody N13 (diluted 1:2000 in assay buffer to a final concentration of 1 µg/ml) under agitation at 700 rpm for 60 minutes. After washing another three times with 300 µL washing buffer, 100 µL of the chromogenic substrate solution 3,3',5,5'-tetramethylbenzidine (TMB, Blue Star, Adaltis, Rome, Italy) was added and MTPs were finally incubated under agitation at 700 rpm for 15-30 minutes. After sufficient color development, the reaction was stopped by adding 50 µL 1 N H₂SO₄, followed by brief agitation and photometrical analysis at a wavelength of 450 nm within 5 minutes after adding stop solution. Afamin concentrations were calculated with a linear regression model (x axis and y axis log transformed) after subtraction of the average blank.

### Example 3 novel ELISA - monoclonal/monoclonal sandwich

This ELISA followed a similar protocol as described for the polyclonal/monoclonal sandwich format (see example 2). The main difference was the use of two monoclonal antibodies (N13 and M14) for sample capture and detection. It turned out, however, that this ELISA protocol only worked when antibody N13 (which is the detection antibody in our previously described sandwich format, see example 2) was used as coating antibody and mab M14 served (as peroxidase conjugate) for detection. Furthermore, the coating antibody N13 is bound to MTPs by simple adhesion to the plastic surface and not via biotin/streptavidin coupling.

In brief, the procedure for the novel double-monoclonal-antibody ELISA follows the protocol indicated below:
Coating of first antibody

MTPs are incubated with 100 microliters of 2.5 microgram/mL N13 in coating buffer for 3 hrs at 37°C, then kept at 4°C until use. After pipetting the antibody, MTPs are covered with plastic foil throughout the whole assay procedure.

Saturation (blocking) of MTP wells to suppress unspecific binding.

Unbound N13 is first removed by 3x washing at RT with washing buffer on a plate washer, followed by blocking with 200 microliters blocking buffer for 30 mins at 37°C.

Incubation of samples, standards and controls

All dilutions are made with assay buffer. Standard series is generated by serially diluting the standard plasma 1:400 to 1:25.600: samples and controls are diluted 1:1000. Samples, standards and controls are applied to MTP and incubated for 1 hour at RT on a plate shaker (700 rpm).

Incubation of POX-conjugated second antibody

After another washing step with 3x applying washing buffer (as above), 100 microliters of POX-conjugated M14, diluted in assay buffer, is added and incubated again for 1 hour at RT on a plate shaker (700 rpm). POX conjugation was performed with Roche's commercially available kit according to the manufacturing protocol. Optimal amounts and dilution were chosen after "checker-board titration".

Addition of substrate, stopping of enzyme reaction, plate reading

After another washing step with 3x applying washing buffer (as above), 100 microliters of substrate solution is applied to each well and incubated in the dark for 30 mins at RT on a plate shaker (700 rpm). Thereafter, the reaction is stopped by adding 50 microliters of stop solution. Read OD at 450 nm within 5 mins with ELISA plate reader.

Buffers and other material
1. MTP: MaxiSorp with flat bottom (Nunc)
2. Coating buffer: PBS, pH 7.3, 1 g/L NaN₃
3. Washing buffer: PBS, pH 7.3, 0.05 vol% Tween-20
4. Blocking/assay buffer: 0.1% casein in PBS pH 7.3
5. POX-substrate: 3,3',5,5'-tetramethylbenzidine (TMB), Blue Star, Adaltis, Rome, Italy)
6. Stop solution: 0.5 M H₂SO₄

### Additional Antibody combinations in sandwich ELISA

In order to compare results obtained with the novel mab N13-mab M14-POX ELISA with further combinations of antibodies, we additionally performed ELISA measurements using the following antibody sandwich design:
a) polyclonal anti-afamin antibody (pab) for coating and mab M14-POX for detection
b) mab M14 for coating and mab N13-POX for detection
c) mab M14 for coating and pab for detection (by means of POX-anti-rabbit antibody as secondary antibody, thus resulting in a triple sandwich ELISA design)
   Commercial afamin ELISA

The commercially available afamin ELISA kit is also of double-antibody sandwich type. MTPs are provided precoated with capture antibody. Detection antibodies are biotinylated and detection occurs with enzyme-conjugated avidin. Neither antibodies nor used standards are characterized; samples have to be diluted 40.000-fold which is unusually high.

### Samples

EDTA-plasma samples for afamin measurements in duplicates from 72 probands (for comparing the various ELISA sandwich combinations) and 8 probands (for comparing our novel ELISA with the commercially available afamin ELISA (Uscn)) out of a population-based human cohort were used.

### Example 4 Results of the different ELISA protocols

### Established ELISA

The afamin assay had a within-run CV_{A} of 8.7% and a total CV_{A} of 9.9% at a mean concentration of 26 mg/L, a within-run CV_{A} of 3.3% and a total CV_{A} of 6.2% at a mean concentration of 73 mg/L and a within-run CV_{A} of 4.3% and a total CV_{A} of 5.4% at a mean concentration of 87 mg/L.

For all further results regarding assay characteristics (biological variation, afamin histogram in healthy blood donors, influences of age, sex, fasting state, differences between plasma and serum, diurnal stability, storage stability, etc) please see the comprehensive analysis reported by Dieplinger et al. (Dieplinger B et al. Clin Chim Acta 2013; 425:236-41). To summarize, afamin concentrations measured by this ELISA turned out to be very robust and independent of storage conditions, fasting state, age, sex and other biological variations.

### Novel ELISA

### Epitope Mapping of mabs N13 and M14

Characterization of the epitope binding of the two monoclonal antibodies indicated that their recognition epitopes are different, non-linear, conformational structural epitopes (see PepSpot data, Table 1, Fig.1). As a consequence, the ratio of available epitope to mass of protein is dependent on retention of the structure of the epitope during afamin purification; this ratio varies with each purification. To compensate for this variation, the originally purified protein was maintained as a primary reference standard. Large amounts of plasma sample aliquots, obtained from fasting healthy blood donors, were used as secondary standard and calibrated to the primary reference standard. Output was defined as measurable afamin in mg per L.

### Comparison of different sandwich designs

The used protocol for the inventive novel, double-monoclonal sandwich ELISA works best when mab N13 is used for coating and mab M14-POX serves as detection antibody (Table 2, Figure 4). Intra-assay CV values were 5.2%, mean values between this assay and the conventional ELISA were in good agreement. When mab M14 was used as coating and mab N13-POX as detection antibody (Table 2, Figure 3), the assay had a substantially inferior performance regarding correlation, linearity and mean value (Table 2, Figure 4). In addition, the afamin ELISA was also performed with pab as coating antibody and mab M14-POX for detection which was working well similar to the originally designed pab/N13 combination. Interestingly, when using mab M14 as coating antibody and pab for detection (followed by an additional incubation with POX anti-rabbit immunoglobulins) the ELISA did not work at all. The colour development in the ELISA plates was extremely intense and could not be evaluated (data not shown).

### Comparison of novel ELISA with commercial ELISA kit

Data from afamin quantifications in 8 plasma samples were compared using the novel ELISA and the commercially available kit from Uscn Life Sciences (Wuhan, China). As can be seen from Table 3 and Figure 5, mean values were only 15% of those obtained from the novel mab/mab ELISA. Furthermore, CV values were also substantially worse as well as the correlation coefficient between these 2 assays (Figure 5).

Within-run and total imprecision CV_{A} of <9% and <10%, respectively, for the established and the novel ELISA was adequate. In addition, the analyte afamin showed good stability data at different storage temperatures. Afamin can therefore be considered stable for 24 h at RT, for 48 h at 4 °C, and for at least one year at -20 °C and at -80 °C. Thus, the analyte is well suitable for routine settings in laboratories, and also provides unproblematic conditions for sample shipment and storage.

When comparing the inventive mab/mab ELISA with the conventional pab/mab N13 ELISA, it was surprising that the inventive ELISA performed substantially better and in agreement with the conventional pab/mab N13 assay. When the second antibody (M14) is used for coating and the first antibody (N13) for detection, the signals were weaker leading to inferior linearity and precision. This effect is caused by the different epitope recognitions of the two monoclonal antibodies (see Table 1, Figure 1).

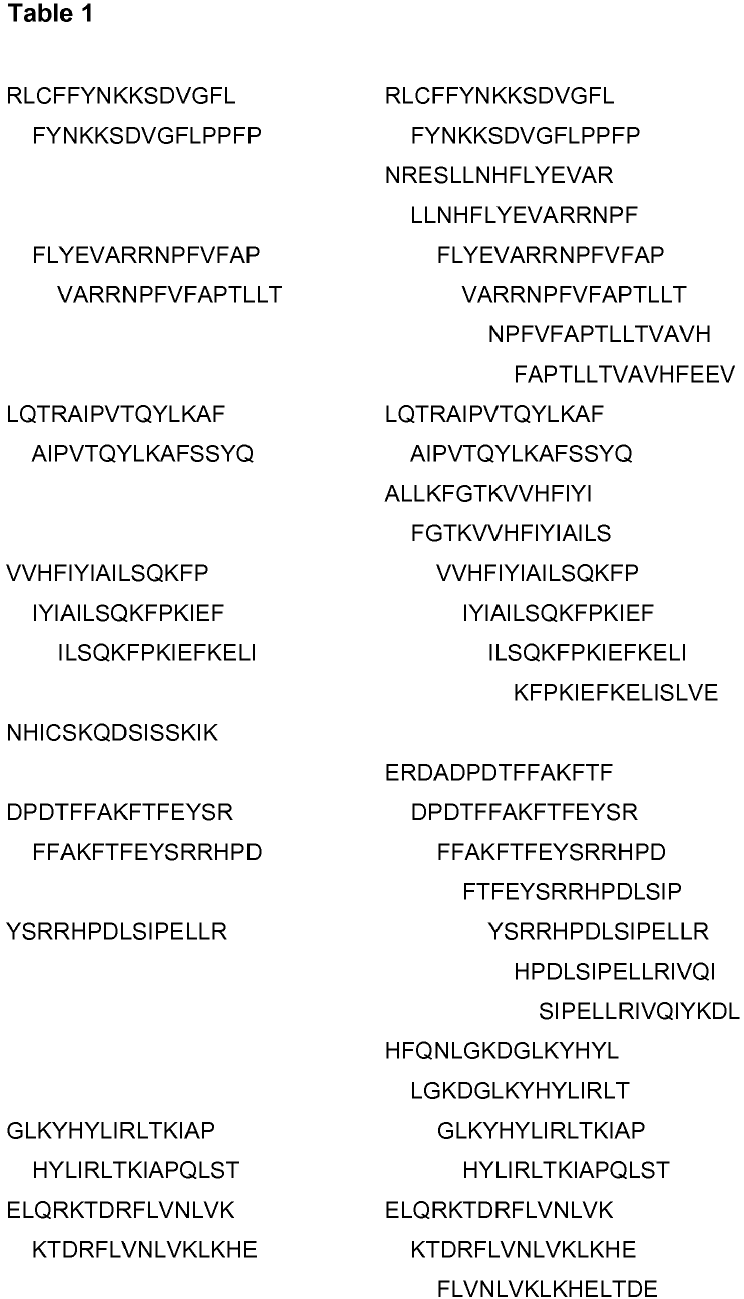

**Table 2**

| **ID** | **Pab/mab N13** | **Pab/mab M14** | **Mab M14/mab N13** | **Mab N13/mab M14** |
|---|---|---|---|---|
| X1 | 100,78 | 115,17 | 71,27 | 118,79 |
| X2 | 84,55 | 119,56 | 83,56 | 96,30 |
| X3 | 89,75 | 105,54 | 95,54 | 96,30 |
| X4 | 90,97 | 123,38 | 107,24 | 98,83 |
| X5 | 90,05 | 110,68 | 103,37 | 98,83 |
| X6 | 98,33 | 119,83 | 102,07 | 96,30 |
| X7 | 108,77 | 128,66 | 138,65 | 118,79 |
| X8 | 101,70 | 146,75 | 98,16 | 131,06 |
| X9 | 124,19 | 121,27 | 82,21 | 122,81 |
| X10 | 84,24 | 97,42 | 58,57 | 75,78 |
| X11 | 90,36 | 104,14 | 67,08 | 88,67 |
| X12 | 85,16 | 99,25 | 76,78 | 88,67 |
| X13 | 64,46 | 81,98 | 62,85 | 78,37 |
| X14 | 76,01 | 85,31 | 67,08 | 78,37 |
| X15 | 69,62 | 77,37 | 69,88 | 80,96 |
| X16 | 68,41 | 78,26 | 48,34 | 78,37 |
| X17 | 68,71 | 93,75 | 40,79 | 83,54 |
| X18 | 55,69 | 74,61 | 42,32 | 59,94 |
| X19 | 61,74 | 67,27 | 39,25 | 57,25 |
| X20 | 62,04 | 71,84 | 54,23 | 62,61 |
| X21 | 63,56 | 64,57 | 42,32 | 57,25 |
| X22 | 61,44 | 66,77 | 46,85 | 70,54 |
| X23 | 85,16 | 101,62 | 64,27 | 91,22 |
| X24 | 72,36 | 91,72 | 57,13 | 98,83 |
| X25 | 84,85 | 103,85 | 46,85 | 103,86 |
| X26 | 76,62 | 105,17 | 43,84 | 70,54 |
| X27 | 69,32 | 82,31 | 57,13 | 80,96 |
| X28 | 56,30 | 103,49 | 82,21 | 98,83 |
| X29 | 102,93 | 103,70 | 86,25 | 106,37 |
| X30 | 109,69 | 108,88 | 75,40 | 123,71 |
| X31 | 112,16 | 102,55 | 79,50 | 113,84 |
| X32 | 96,49 | 111,00 | 64,27 | 98,83 |
| X33 | 65,07 | 69,31 | 36,14 | 80,96 |
| X34 | 55,09 | 61,91 | 39,25 | 65,27 |
| X35 | 56,60 | 59,22 | 37,70 | 59,94 |
| X36 | 57,50 | 60,77 | 45,35 | 54,55 |
| X37 | 103,54 | 115,34 | 76,78 | 126,17 |
| X38 | 82,11 | 105,61 | 87,59 | 83,19 |
| X39 | 119,86 | 111,40 | 80,86 | 125,20 |
| X40 | 116,16 | 103,99 | 69,88 | 135,93 |
| X41 | 117,40 | 107,73 | 75,40 | 164,74 |
| X42 | 75,40 | 78,79 | 46,85 | 80,96 |
| X43 | 71,14 | 69,73 | 45,35 | 75,78 |
| X44 | 76,01 | 78,89 | 55,68 | 73,17 |
| X45 | 71,75 | 68,38 | 49,83 | 86,11 |
| X46 | 80,58 | 78,52 | 52,77 | 88,67 |
| X47 | 93,42 | 74,76 | 52,77 | 78,37 |
| X48 | 75,40 | 75,01 | 54,23 | 73,17 |
| X49 | 84,24 | 86,35 | 48,34 | 80,96 |
| X50 | 74,49 | 78,84 | 40,79 | 78,37 |
| X51 | 81,50 | 78,00 | 45,35 | 80,96 |
| X52 | 74,18 | 72,18 | 45,35 | 83,54 |
| X53 | 64,46 | 73,06 | 46,85 | 80,96 |
| X54 | 82,72 | 76,18 | 48,34 | 80,96 |
| X55 | 68,41 | 66,59 | 45,35 | 75,78 |
| X56 | 55,39 | 75,62 | 42,32 | 75,78 |
| X57 | 21,48 | 41,43 | 52,77 | 21,35 |
| X58 | 61,44 | 77,01 | 46,85 | 67,91 |
| X59 | 82,72 | 86,35 | 49,83 | 73,17 |
| X60 | 89,13 | 96,82 | 55,68 | 96,30 |
| X61 | 86,69 | 96,82 | 54,23 | 83,54 |
| X62 | 83,94 | 94,39 | 60,01 | 93,77 |
| X63 | 89,75 | 90,36 | 67,08 | 91,22 |
| X64 | 45,76 | 48,95 | 46,85 | 62,61 |
| X65 | 50,87 | 63,64 | 36,14 | 46,35 |
| X66 | 56,30 | 49,87 | 31,37 | 88,67 |
| X67 | 45,46 | 48,16 | 31,37 | 67,91 |
| X68 | 51,17 | 67,73 | 37,70 | 57,25 |
| X69 | 100,47 | 95,43 | 67,08 | 108,86 |
| X70 | 97,10 | 94,59 | 72,65 | 103,86 |
| X71 | 55,99 | 93,75 | 62,85 | 86,11 |
| X72 | 106,93 | 114,33 | 64,27 | 93,77 |
| **Mean** | **79,08** | **88,24** | **60,57** | **86,91** |

**Table 3**

| **sample nr** | **Mab N13/mab M14** | **CV %** | **Uscn** | **CV %** |
|---|---|---|---|---|
| 292 | 120,99 | 9,04 | 23,56 | 16,99 |
| 299 | 101,35 | 3,76 | 12,40 | 11,58 |
| 300 | 95,84 | 4,74 | 22,72 | 6,17 |
| 301 | 71,38 | 4,89 | 16,32 | 3,90 |
| 302 | 89,37 | 5,23 | 12,60 | 12,71 |
| 303 | 103,22 | 6,98 | 7,60 | 14,74 |
| 304 | 106,61 | 3,23 | 12,28 | 30,78 |
| 305 | 90,79 | 0,87 | 13,92 | 4,26 |
| **mean** | **97,44** | **4,84** | **15,18** | **12,64** |

## Claims

1. A method for the detection and/or quantification of afamin in a sample comprising
• providing a carrier surface with an immobilized first antibody against afamin,
• contacting a sample which potentially comprises afamin with the first antibody,
• providing a second antibody against afamin
• detecting binding events of afamin, wherein the second antibody has the means to allow detection and/or quantification of binding events by visual inspection and/or spectrophotometry,
**characterised in that** the first and the second antibody are monoclonal antibodies and bind to different epitopes of afamin.

2. Method according to claim 1, wherein the first or the second antibody, preferably the first antibody, binds to an epitope which includes amino acids Arg155, Leu227, and Phe419 of afamin.

3. Method according to claim 1 or 2, wherein the first or the second antibody, preferably the second antibody, binds to an epitope which includes amino acids Leu202, Ile288, and Lys300 of afamin.

4. Method according to any one of the preceding claims, **characterised in that** the first and the second antibodies bind to different conformational epitopes.

5. Method according to any one of the preceding claims, **characterised in that** the second antibody is an enzyme labelled, fluorescence labelled, or dye labelled antibody.

6. Method according to any one of the preceding claims, **characterised in that** the second antibody is an enzyme labelled antibody.

7. Method according to claim 6, **characterised in that** the enzyme is horse-radish peroxidase.

8. Method according to claim 6, **characterised in that** a chromogenic substrate solution is provided for detecting binding events.

9. Method according to claim 7, **characterised in that** the chromogenic substrate solution comprises 3,3',5,5'-tetramethylbenzidine.

10. Method according to any one of the preceding claims, **characterised in that** the carrier is a solid surface or a membrane.

11. Method according to any one of the preceding claims, **characterised in that** the carrier is a microtiter plate.

12. Method according to any one of the preceding claims, **characterised in that** the first antibody is immobilized on the carrier by adhesion.

13. Method according to any one of the preceding claims, **characterised in that** the sample is a sample of body fluid or a tissue sample.

14. Method according to claim 13, **characterised in that** the sample is a blood, blood plasma or blood serum sample.

15. Kit for performing the method according to any one of claims 1 to 14, **characterised in that** it comprises reference means of afamin.

16. Kit according to claim 15, **characterised in that** the reference means is a standardized amount of afamin.

17. Kit according to claim 15 or 16, **characterised in that** the reference means is a series of standardized samples of afamin.

18. Use of a kit according to claims 15 to 17, **characterised in that** the content of afamin determined in the sample is compared with a reference value.
